# EUROPÄISCHE PATENTSCHRIFT

(11) **EP 3 669 814 B1**
(45) Veröffentlichungstag und Bekanntmachung des Hinweises auf die Patenterteilung: **21.10.2020**
(21) Anmeldenummer: 18214291.9
(22) Anmeldetag: 20.12.2018
(51) Int. Cl.: A61C 1/00, A61B 90/98

(54) **MEDIZINISCHES ODER DENTALES INSTRUMENT MIT EINEM RFID-SPEICHERETIKETT**
MEDICAL OR DENTAL INSTRUMENT WITH A RFID STORAGE LABEL
INSTRUMENT MÉDICAL OU DENTAIRE DOTÉ D'UNE ÉTIQUETTE MÉMOIRE RFID

(43) Veröffentlichungstag der Anmeldung: 24.06.2020
(73) Patentinhaber: W & H Dentalwerk Bürmoos GmbH, 5111 Bürmoos (AT)
(72) Erfinder: PRUCKNER, Christian, 1190 Wien (AT); SPITZAUER, Josef, 5110 Loipferding (AT); REITER, Michael, Dr., 5061 Elsbethen (AT)
(74) Vertreter: Benda, Ralf

(56) Entgegenhaltungen:
- WO-A1-2014/017530
- WO-A1-2015/149614
- WO-A1-2018/167373
- WO-A1-2018/195615
- US-A1- 2008 106 419

## Beschreibung

Die vorliegende Erfindung betrifft ein medizinisches oder dentales Instrument mit einem RFID-Speicheretikett sowie ein entsprechendes Verfahren zur Herstellung oder zum Nachrüsten eines derartigen medizinischen oder dentalen Instruments.

Aus dem Stand der Technik, zum Beispiel der Anmeldeschrift US 2010/0219252 A1, ist es bekannt, ein RFID-Speicheretikett mit einem Speicherchip elektrisch mit einem metallischen Bauteil einer Vorrichtung, zum Beispiel einer Getränkedose, derart zu verbinden, dass das metallische Bauteil als Antenne für das RFID-Speicheretikett zur drahtlosen Energie- und/ oder Datenübertragung mit einer entfernten Lese- und/ oder Schreibvorrichtung dient. Dadurch ist es vorteilhafterweise möglich, die Abmessungen des RFID-Speicheretiketts zu verkleinern, da das RFID-Speicheretikett nur eine - im Vergleich zu herkömmlichen RFID-Speicheretiketten - verkleinerte Antenne benötigt.

Die Patentanmeldung US 2008/0106419 A1 offenbart ein RFID-Etikett, bei dem zwei Antennenabschnitte durch eine elektrisch leitendes Klebemittel galvanisch miteinander verbunden sind.

Die Anmeldeschrift WO 2018/195615 A1 beschreibt chirurgische Instrumente mit RFID-Etiketten, die mittels eines metallischen Halters an dem chirurgischen Instrument verschweißt sind, um eine induktive Energie- und Datenübertragen zu gewährleisten.

Der vorliegenden Erfindung liegt die Aufgabe zugrunde, die Energie- und/ oder Datenübertragung zwischen dem metallischen, die Antenne bildenden Bauteil und dem RFID-Speicheretikett zu verbessern, insbesondere, wenn das metallische, die Antenne bildende Bauteil Teil eines medizinischen oder dentalen Instruments ist.

Diese Aufgabe wird gemäß der vorliegenden Erfindung durch ein medizinisches oder dentales Instrument mit den Merkmalen des Anspruchs 1 und durch ein Verfahren zur Herstellung oder zum Nachrüsten eines derartigen medizinischen oder dentalen Instruments mit den Merkmalen des Anspruchs 14 gelöst. Besonders vorteilhafte Ausführungsformen sind in den Unteransprüchen angeführt.

Das medizinische oder dentale Instrument umfasst ein metallisches Instrumenten-Bauteil und ein RFID-Speicheretikett mit einem Speicherchip zur Speicherung von Daten, wobei das RFID-Speicheretikett derart an dem medizinischen oder dentalen Instrument, insbesondere an dem metallischen Instrumenten-Bauteil befestigt ist, dass das RFID-Speicheretikett mit dem metallischen Instrumenten-Bauteil elektrisch verbunden ist, so dass das metallische Instrumenten-Bauteil eine Antenne für das RFID-Speicheretikett zur drahtlosen Energie- und/ oder Datenübertragung mit einer entfernten Lese- und/ oder Schreibvorrichtung bildet. Das medizinische oder dentale Instrument umfasst des Weiteren ein elektrisch leitendes Koppelelement, welches das RFID-Speicheretikett elektrisch mit dem eine Antenne bildenden, metallischen Instrumenten-Bauteil verbindet, wobei das elektrisch leitende Koppelelement eine im Wesentlichen plane, erste Koppelfläche, auf welcher das RFID-Speicheretikett angeordnet ist, und eine zweite Koppelfläche aufweist, welche an dem eine Antenne bildenden, metallischen Instrumenten-Bauteil angeordnet ist.

Ein entsprechendes Verfahren zur Herstellung oder zum Nachrüsten eines medizinischen oder dentalen Instruments umfasst:
- das zur Verfügung Stellen eines medizinischen oder dentalen Instruments mit einem metallischen Instrumenten-Bauteil,
- das zur Verfügung Stellen eines RFID-Speicheretiketts mit einem Speicherchip zur Speicherung von Daten,
- das Befestigen des RFID-Speicheretiketts an dem medizinischen oder dentalen Instrument, insbesondere an dem metallischen Instrumenten-Bauteil mittels eines elektrisch leitenden Koppelelements, derart, dass das RFID-Speicheretikett mit dem metallischen Instrumenten-Bauteil elektrisch verbunden ist, so dass das metallische Instrumenten-Bauteil eine Antenne für das RFID-Speicheretikett zur drahtlosen Energie- und/ oder Datenübertragung mit einer entfernten Lese- und/ oder Schreibvorrichtung bildet, wobei das elektrisch leitende Koppelelement, eine im Wesentlichen plane, erste Koppelfläche, auf welcher das RFID-Speicheretikett angeordnet ist, und eine zweite Koppelfläche aufweist.

In praktischen Versuchen konnte durch das Vorsehen eines elektrisch leitenden Koppelelements, das eine im Wesentlichen plane, erste Koppelfläche, auf welcher das RFID-Speicheretikett angeordnet ist, und eine zweite Koppelfläche aufweist, welche an dem eine Antenne bildenden, metallischen Instrumenten-Bauteil angeordnet ist, eine verbesserte bzw. ausgezeichnete Energie- und/ oder Datenübertragung festgestellt werden. Ohne sich an eine bestimmte Annahme zu binden wird vermutet, dass das elektrisch leitenden Koppelelement, insbesondere das Vorsehen der planen, ersten Koppelfläche und der zweiten Koppelfläche, die auf (verlustbehafteten) kapazitiven Effekten beruhende Energie- und/ oder Datenübertragung zwischen dem RFID-Speicheretikett und dem metallischen Instrumenten-Bauteil positiv beeinflusst. Die plane, erste Koppelfläche bildet insbesondere in vorteilhafter Weise eine optimale Kontakt- oder Übertragungsfläche für die Energie- und/ oder Datenübertragung.

Vorzugsweise weist das metallische Instrumenten-Bauteil eine erste Biegung und die zweite Koppelfläche des elektrisch leitenden Koppelelements eine zur ersten Biegung des metallischen Instrumenten-Bauteils komplementäre zweite Biegung auf, wobei zur elektrischen Verbindung des RFID-Speicheretiketts mit dem eine Antenne bildenden, metallischen Instrumenten-Bauteil die erste Biegung und die zweite Biegung ineinandergefügt sind.

Das Verfahren zur Herstellung oder zum Nachrüsten eines medizinischen oder dentalen Instruments umfasst vorzugsweise, dass das metallische Instrumenten-Bauteil eine erste Biegung und die zweite Koppelfläche des elektrisch leitenden Koppelelements eine zur ersten Biegung des metallischen Instrumenten-Bauteils komplementäre zweite Biegung aufweist, wobei zur elektrischen Verbindung des RFID-Speicheretiketts mit dem eine Antenne bildenden, metallischen Instrumenten-Bauteil die erste Biegung und die zweite Biegung ineinander gefügt werden.

Die zweite Koppelfläche mit einer zweiten Biegung zu formen, die eine zur ersten Biegung des metallischen Instrumenten-Bauteils komplementäre Form hat, bewirkt in vorteilhafter Weise eine deutliche verbesserte Übertragung oder Einkopplung von Energie und/ oder Daten zwischen dem RFID-Speicheretikett und dem als Antenne wirkenden, gebogenen, metallischen Instrumenten-Bauteil, da durch die zweite, gebogene Koppelfläche eine vergrößerte Kontaktfläche oder Übertragungsfläche für Energie und/ oder Daten zwischen dem (flach ausgebildeten) RFID-Speicheretikett und dem gebogenen metallischen Instrumenten-Bauteil gebildet ist. Dies ist insbesondere bei medizinischen oder dentalen Instrumenten, die eine Vielzahl von gebogenen Instrumenten-Bauteilen aufweisen, von großem Vorteil. Ein derartiges elektrisch leitendes Koppelelement mit einer gebogenen zweiten Koppelfläche ermöglicht insbesondere auch eine einfache Nachrüstung eines medizinischen oder dentalen Instruments, bei dem das (im Wesentlichen mit planen Außenseiten ausgebildete) RFID-Speicheretikett mit Hilfe des elektrisch leitenden Koppelelements mit einer gebogenen zweiten Koppelfläche an der gebogenen Außen- oder Griffhülse des medizinischen oder dentalen Instruments anordenbar oder befestigbar ist. Das medizinische oder dentale Instrument ist hierbei vorzugsweise als gerades Handstück oder gewinkeltes Winkelstück ausgebildet.

Vorzugsweise sind/ werden die erste Biegung und die zweite Biegung im Wesentlichen abstandslos und/ oder berührend ineinandergefügt. Vorzugsweise sind die erste Biegung und die zweite Biegung einander geometrisch, insbesondere in ihrer dreidimensionalen Form ergänzend ausgebildet. Vorzugsweise sind die erste Biegung und die zweite Biegung derart geformt, dass eine der beiden Biegungen konkav und die andere der beiden Biegungen konvex geformt ist. Diese Ausgestaltungen bewirken in vorteilhafter Weise eine zusätzlich Verbesserung der Energie- und/ oder Datenübertragung.

Das medizinische oder dentale Instrument ist insbesondere als ein mit einer Hand haltbares Instrument ausgebildet. Das medizinische oder dentale Instrument ist insbesondere als Diagnose und/ oder Behandlungsinstrument ausgebildet. Das medizinische oder dentale Instrument umfasst vorzugsweise zumindest eines der folgenden Elemente: ein gerades oder gebogenes oder pistolenförmiges Handstück; ein Winkelstück, an dem ein Werkzeug gewinkelt zu einer Längsachse eines Griffabschnitts befestigbar ist; ein Handgriffelement; ein Instrument, welches vorgesehen ist, elektromagnetische Strahlung abzugeben; ein Instrument, welches vorgesehen ist, ein Fluid abzugeben; ein Instrument, welches vorgesehen ist, ein diagnostisches und/ oder therapeutisches Medium abzugeben; ein Instrument, welches vorgesehen ist, Schwingungen, insbesondere Ultraschallschwingungen abzugeben; eine Motoreinheit zum Antrieb eines Werkzeugs, insbesondere mit einem pneumatisch oder elektrisch betriebenen Motor; ein Instrument mit einer Bildaufnahmevorrichtung, insbesondere mit einer Kamera; ein Kupplungselement zur Verbindung mit einer Medienquelle, insbesondere einer Flüssigkeitsquelle, einer Gasquelle oder einer Quelle von elektrischer Energie; eine Adaptereinheit.

Das RFID-Speicheretikett weist bevorzugt einen (kurzen) Antennenabschnitt auf, der insbesondere (nur) durch Zusammenwirken mit dem als Antenne wirkenden, metallischen Instrumenten-Bauteil eine ausreichende Lese- und/ oder Sendereichweite zur Energie- und/ oder Datenübertragung mit der entfernten Lese- und/ oder Schreibvorrichtung erzielt, wodurch in vorteilhafter Weise die Abmessungen des RFID-Speicheretiketts und somit der Platzbedarf am oder im medizinischen oder dentalen Instrument verringert sind.

Vorzugsweise bildet das RFID-Speicheretikett vor der Befestigung am oder im medizinischen oder dentalen Instrument eine eigenständig und/ oder vorgefertigte Baueinheit. Vorzugsweise bilden das RFID-Speicheretikett und das medizinische oder dentale Instrument eigenständige und/ oder vorgefertigte Baueinheiten, insbesondere vor der Befestigung des RFID-Speicheretiketts an dem medizinischen oder dentalen Instrument. Vorzugsweise bilden das RFID-Speicheretikett und das medizinische oder dentale Instrument nach der Befestigung des RFID-Speicheretiketts an dem medizinischen oder dentalen Instrument eine operative, insbesondere kommunikative Einheit. Das eigenständige, vorgefertigte RFID-Speicheretikett erleichtert in vorteilhafter Weise dessen Montage oder Befestigung an dem medizinischen oder dentalen Instrument.

Vorzugsweise ist/ wird das RFID-Speicheretikett mit zumindest einem der folgenden Mittel am medizinischen oder dentalen Instrument angeordnet oder befestigt: einem Klebemittel; einem Verguss- oder Spritzmaterial, insbesondere aus Kunststoff; einem Lötmaterial; einem mechanisch mit dem medizinischen oder dentalen Instrument verbindbaren Element. Das mechanische Element umfasst zum Beispiel einen Verbindungsring, an dem das RFID-Speicheretikett vorgesehen ist und der über einen Abschnitt, zum Beispiel einen Endabschnitt oder Kupplungsabschnitt, des medizinischen oder dentalen Instruments aufschiebbar ist. Das mechanische Element ist vorzugsweise aus elektrisch nicht leitendem Material, zum Beispiel Kunststoff oder Keramik gebildet, um eine Beeinflussung der Energie- und/ oder Datenübertragung zu oder von dem RFID-Speicheretikett zu verhindern. Die genannten Mittel ermöglichen in vorteilhafterweise eine einfach und rasche Befestigung und/ oder Nachrüstung des RFID-Speicheretiketts an dem medizinischen oder dentalen Instrument.

Die Datenübertragung zwischen dem RFID-Speicheretikett und der entfernten Lese- und/ oder Schreibvorrichtung kann uni- oder bidirektional ausgebildet sein. Der Speicherchip des RFID-Speicheretiketts ist somit entweder als "nur-Lese-Speicher" (ROM-Speicher), von dem Daten nur ausgelesen werden können (unidirektionale Datenübertragung), oder als "Schreib-Lese-Speicher", der wiederholt mit Daten beschrieben werden kann und von dem Daten ausgelesen werden können, ausgebildet (bidirektionale Datenübertragung).

Vorzugsweise ist das metallische Instrumenten-Bauteil, insbesondere mit der ersten Biegung, zylindrisch oder hohl-zylindrisch geformt, wobei die erste Biegung insbesondere durch die Krümmung der Mantelfläche des zylindrischen oder hohl-zylindrischen metallischen Instrumenten-Bauteils gebildet ist. Alternativ oder zusätzlich ist das metallische Instrumenten-Bauteil, insbesondere mit der ersten Biegung, hülsenförmig ausgebildet oder umfasst eine Hülse des medizinischen oder dentalen Instruments. Vorzugsweise ist das hülsenförmige, metallische Instrumenten-Bauteil zumindest ein (metallischer) Abschnitt einer Griffhülse oder einer Außenhülse oder einer Lagerhülse für ein Element, zum Beispiel eine Welle, des medizinischen oder dentalen Instruments oder einer Kupplungshülse zur lösbaren Verbindung des medizinischen oder dentalen Instruments mit einem Kupplungsteil, zum Beispiel mit einem Werkzeug, Motor oder Adapter. Vorzugsweise ist das metallische Instrumenten-Bauteil rohr- oder leitungsförmig ausgebildet. Damit ist es in vorteilhafter Weise möglich, ein vorhandenes Bauteil des medizinischen oder dentalen Instruments als Träger für das RFID-Speicheretikett, insbesondere auch zum Nachrüsten, zu verwenden.

Vorzugsweise ist das metallische Instrumenten-Bauteil flach ausgebildet oder weist eine plane Fläche auf, auf welcher das RFID-Etikett angeordnet ist. Das flache metallische Instrumenten-Bauteil ist zum Beispiel als Wand(abschnitt) im Inneren des medizinischen oder dentalen Instruments oder als Betätigungselement, bevorzugt als Taster- oder Druckdeckel, insbesondere zum Betätigen einer Werkzeughaltevorrichtung, ausgebildet. Damit ist es in vorteilhafter Weise möglich, ein vorhandenes Bauteil des medizinischen oder dentalen Instruments als Träger für das RFID-Speicheretikett, insbesondere auch zum Nachrüsten, zu verwenden.

Vorzugsweise ist das RFID-Speicheretikett an der Außenseite des medizinischen oder dentalen Instruments, besonders bevorzugt an der Außenseite des metallischen Instrumenten-Bauteils, welches insbesondere eine erste Biegung aufweist, insbesondere an der Außenseite der Griffhülse oder der Außenhülse angeordnet. Damit ist in vorteilhafter Weise eine einfache Befestigung und/ oder Nachrüstung des medizinischen oder dentalen Instruments mit dem RFID-Speicheretikett möglich. Auch ist die Griff- oder Außenhülse aufgrund ihrer Länge in vorteilhafter Weise besonders gut als Antenne verwendbar und ermöglicht damit eine besonders zuverlässige Energie- und/ oder Datenübertragung.

Die, insbesondere zylindrische oder hohlzylindrische, Griff- oder Außenhülse ist bevorzugt ein- oder mehrteilig ausgebildet. Die Griff- oder Außenhülse ist vorzugsweise rohrförmig ausgebildet und/ oder weist einen hohlen Innenraum auf, insbesondere zur Aufnahme einer oder mehrerer Bauteile, zum Beispiel von Teilen der Antriebsvorrichtung und/ oder einer Medien-, Versorgungs- und/ oder Datenleitung des medizinischen oder dentalen Instruments. Die Griff- oder Außenhülse umfasst (entlang ihrer Längsachse) vorzugsweise zumindest zwei gewinkelt (mit einem Winkel >0°) oder gebogen zueinander angeordnete Abschnitte.

Vorzugsweise ist das RFID-Speicheretikett im Inneren des medizinischen oder dentalen Instruments, insbesondere eines Handstücks oder Winkelstücks angeordnet, zum Beispiel an einer Lagerhülse für ein Element des medizinischen oder dentalen Instruments oder an einer Kupplungshülse des medizinischen oder dentalen Instruments. Vorzugsweise ist das RFID-Speicheretikett an einem im Inneren des medizinischen oder dentalen Instruments vorgesehenen Bauteils angeordnet oder befestigt. Damit ist das RFID-Speicheretikett besonders gut vor äußeren Einflüssen, zum Beispiel Verschmutzungen oder Feuchtigkeit, geschützt.

Das elektrisch leitende Koppelelement bildet mit zumindest Teilen des RFID-Speicheretiketts und des eine Antenne bildenden, metallischen Instrumenten-Bauteils, insbesondere in einem Ersatzschaltbild, einen elektrischen Kondensator zur kapazitiven Energie- und/ oder Datenübertragung. Demgemäß umfasst das Verfahren zur Herstellung oder zum Nachrüsten eines medizinischen oder dentalen Instruments, dass durch das Befestigen oder Verbinden des elektrisch leitenden Koppelelements und des RFID-Speicheretiketts an oder mit dem eine Antenne bildenden, metallischen Instrumenten-Bauteil ein elektrischer Kondensator zur kapazitiven Energie- und/ oder Datenübertragung gebildet wird. Der Kondensator ermöglicht in vorteilhafter Weise eine drahtlose Übertragung von Energie- und/ oder Daten von und/ oder zu dem RFID-Speicheretikett.

Vorzugsweise umfasst das elektrisch leitende Koppelelement ein elektrisch leitendes Klebemittel, welches das RFID-Speicheretikett mit dem eine Antenne bildenden, metallischen Instrumenten-Bauteil, insbesondere unlösbar, haftend und elektrisch verbindet. Das RFID-Speicheretikett ist somit insbesondere auf das medizinische oder dentale Instrument, vorzugsweise auf das metallische Instrumentenbauteil, aufgeklebt. Das elektrisch leitende Klebemittel umfasst vorzugsweise zumindest eine polymere Verbindung und/ oder zumindest eine organische Verbindung und/ oder zumindest eine Silikonverbindung. Vorzugsweise sind in dem elektrisch leitenden Klebemittel elektrisch leitende Partikel vorgesehen und/ oder dispergiert, zum Beispiel metallische Partikel, insbesondere Kupfer-, Silber- oder Goldpartikel. Vorzugsweise ist das elektrisch leitende Klebemittel (vor der Schaffung der haftenden Verbindung zwischen dem RFID-Speicheretikett und dem metallischen Instrumenten-Bauteil) flüssig oder verflüssigbar. Vorzugsweise ist das elektrisch leitende Klebemittel aushärtbar oder härtet nach der Verbindung zwischen dem RFID-Speicheretikett und dem metallischen Instrumenten-Bauteil aus. Vorzugsweise bilden sich die erste und zweite, insbesondere gebogene, Koppelfläche nach dem Auftragen des elektrisch leitenden Klebemittels auf das RFID-Speicheretikett und/ oder das metallische Instrumenten-Bauteil durch das Kontaktieren oder Anlegen des RFID-Speicheretiketts und des metallischen Instrumenten-Bauteils aneinander und/ oder während des Härtens. Die Verwendung eines elektrisch leitenden Klebemittels ist besonders vorteilhaft, da damit sowohl die elektrische Verbindung zwischen dem RFID-Speicheretikett und dem metallischen Instrumenten-Bauteil als auch die Befestigung des RFID-Speicheretiketts an dem medizinischen oder dentalen Instrument verwirklichbar sind.

Vorzugsweise weist das elektrisch leitende Koppelelement ein elektrisch leitendes Formelement auf. Das elektrisch leitende Formelement weist eine im Wesentlichen plane, erste Koppelfläche, auf welcher das RFID-Speicheretikett angeordnet ist, und eine zweite Koppelfläche auf, welche insbesondere eine zur ersten Biegung des metallischen Instrumenten-Bauteils komplementäre zweite Biegung hat. Das elektrisch leitende Formelement ist bevorzugt durch ein vollständig metallisches Formelement oder durch ein Trägermaterial, zum Beispiel Kunststoff oder Keramik, mit darin angeordneten und/ oder dispergierten metallischen Partikeln gebildet. Die Verwendung eines (festen) Formelements als elektrisch leitendes Koppelelement erleichtert in vorteilhafter Weise die Montage, insbesondere die Verbindung des Koppelelements mit dem RFID-Speicheretikett und/ oder mit dem metallischen Instrumenten-Bauteil.

Das elektrisch leitende Formelement ist/ wird vorzugsweise mit einem Klebemittel an dem medizinischen oder dentalen Instrument, insbesondere an dem metallischen Instrumenten-Bauteil befestigt. Das Klebemittel enthält wahlweise elektrisch leitende Partikel (wie im Vorstehenden beschrieben) oder es enthält keine elektrisch leitende Partikel. Alternativ oder zusätzlich ist das elektrisch leitende Formelement durch eine im Folgenden noch detaillierter beschriebene Kappe, welche das RFID-Speicheretikett umgibt, an dem medizinischen oder dentalen Instrument, insbesondere an dem metallischen Instrumenten-Bauteil befestigt. Die Kappe ist zum Beispiel durch Vergießen oder Verspritzen des RFID-Speicheretiketts gebildet, wobei das elektrisch leitende Formelement mit dem RFID-Etikett vergossen oder verspritzt ist/ wird und/ oder durch das Formen der Kappe durch Vergießen oder Verspritzen des RFID-Speicheretiketts das elektrisch leitende Formelement an der Kappe befestigt ist/ wird. Es ist auch denkbar, das elektrisch leitende Formelement durch Klemmen zwischen der Kappe und dem medizinischen oder dentalen Instrument, insbesondere dem metallischen Instrumenten-Bauteil an dem Instrument oder Bauteil zu befestigen. Die genannten Befestigungen ermöglichen in vorteilhafter Weise eine einfache und rasche Montage des elektrisch leitenden Koppelelements, insbesondere gemeinsam mit der RFID-Speicheretikett. Es ist auch möglich, die Kappe als separates Bauteil zu fertigen und erst bei der Befestigung des RFID-Speicheretiketts an dem medizinischen oder dentalen Instrument das RFID-Speicheretikett mit der Kappe zu verbinden, zum Beispiel einzuschieben.

Vorzugsweise ist das elektrisch leitende Koppelelement, insbesondere das elektrisch leitende Formelement plattenförmig ausgebildet und/ oder weist zumindest eine gewölbte Fläche auf, insbesondere eine konkav oder konvex gewölbte Fläche. Vorzugsweise weist das, insbesondere plattenförmige und/ oder durch ein Klebemittel gebildete, elektrisch leitende Koppelelement zwei einander gegenüberliegende Grundflächen auf, die durch mehrere Seitenflächen verbunden sind. Besonders bevorzugt ist zumindest eine der Grundflächen plan, insbesondere jene Grundfläche, auf welcher das RFID- Speicheretikett angeordnet ist. Besonders bevorzugt ist zumindest eine der Grundflächen gewölbt, insbesondere jene Grundfläche, welche näher zu dem metallischen Instrumenten-Bauteil angeordnet ist oder dieses kontaktiert. Vorzugsweise verläuft die Wölbung von einer der Seitenflächen in Richtung einer Mittellinie der zumindest einen gewölbten Grundfläche, wodurch die Wandstärke des Formelements in Richtung der Mittellinie abnimmt und insbesondere mittig am geringsten ist. Die beschriebene Form des elektrisch leitenden Koppelelements bildet in vorteilhafter Weise eine stabile Verbindung des RFID-Speicheretiketts mit dem metallischen Instrumenten-Bauteil und ermöglicht somit eine zuverlässige Energie- und/ oder Datenübertragung. Insbesondere vergrößert das plattenförmig ausgebildete, elektrisch leitende Koppelelement in vorteilhafter Weise die Kontakt- oder Kopplungsfläche zwischen dem RFID- Speicheretikett und dem metallischen Instrumenten-Bauteil und verbessert somit die Energie- und/ oder Datenübertragung.

Vorzugsweise umfasst das RFID-Speicheretikett zumindest eines der folgenden Elemente: einen, insbesondere keramischen Träger für den Speicherchip; zumindest einen mit dem Speicherchip elektrisch verbundenen elektrischen Leiter, zum Beispiel eine Leiterbahn oder eine elektrisch leitende Beschichtung; eine elektrisch isolierende Schutzschicht; einen Antennenabschnitt, der insbesondere durch den keramischen Träger und den mit dem Speicherchip elektrisch verbundenen elektrischen Leiter verbunden ist. Vorzugsweise umgibt die elektrisch isolierende Schutzschicht den Speicherchip, den Träger für den Speicherchip und den mit dem Speicherchip elektrisch verbundenen elektrischen Leiter.

Vorzugsweise ist das RFID-Speicheretikett scheibenförmig, zylindrisch oder quaderförmig geformt. Vorzugsweise ist das RFID-Speicheretikett ausgebildet, Daten in einem Frequenzbereich von etwa 850 MHz - 980 MHz oder von etwa 2,30 GHz - 2,60 GHz zu übertragen.

Vorzugsweise ist an dem medizinischen oder dentalen Instrument, insbesondere an dem metallischen Instrumenten-Bauteil eine Kappe befestigt, welche das RFID-Speicheretikett umschließt. Besondere bevorzugt kontaktiert und/ oder umschließt die Kappe auch das elektrisch leitende Koppelelement. Vorzugsweise ist die Kappe derart an dem metallischen Instrumenten-Bauteil befestigt, dass das RFID-Speicheretikett und insbesondere auch das elektrisch leitende Koppelelement dichtend umschlossen sind, so dass das Eindringen von Fremdstoffen von außen in das RFID-Speicheretikett unterbunden ist. Vorzugsweise ist die Kappe aus einem elektrisch nicht leitenden Material gefertigt. Vorzugsweise ist die Kappe aus Kunststoff oder Keramik oder einem Klebemittel gefertigt. Die Kappe ist insbesondere durch Vergießen oder Verspritzen gebildet, wobei bevorzugt zur Herstellung der Kappe das RFID-Speicheretikett und gegebenenfalls das elektrisch leitende Koppelelement mit einem Klebemittel oder Kunststoffmaterial vergossen oder verspritzt werden, wodurch besonders bevorzugt das RFID-Speicheretikett und gegebenenfalls das elektrisch leitende Koppelelement an dem medizinischen oder dentalen Instrument, insbesondere an dem metallischen Instrumenten-Bauteil befestigt werden. Vorzugsweise ist das Material zur Bildung der Kappe vor dem Vergießen oder Verspritzen flüssig und härtet nach dem Vergießen oder Verspritzen aus. Vorzugsweise umfassen das elektrisch leitende Koppelelement und die Kappe dasselbe Klebemittel, jedoch enthält nur das Klebemittel des elektrisch leitenden Koppelelements elektrisch leitende Partikel und/ oder es enthält das die Kappe bildende Klebemittel keine elektrisch leitende Partikel.

Die Erfindung wird nachfolgend anhand bevorzugter Ausführungsbeispiele und Bezug nehmend auf die beigefügten Zeichnungen erläutert.
Figur 1 zeigt ein medizinisches oder dentales Instrument mit einem RFID-Speicheretikett und einem metallischen Instrumenten-Bauteil, das eine Antenne für das RFID-Speicheretikett bildet;
Figur 2 zeigt eine Schnittansicht durch ein RFID-Speicheretikett, das auf einem metallischen Instrumenten-Bauteil, das eine Antenne für das RFID-Speicheretikett bildet, angeordnet ist;
Figur 3 zeigt Hauptkomponenten eines RFID-Speicheretiketts einschließlich eines elektrisch leitenden Kopplungselements zum elektrischen Verbinden des RFID-Speicheretiketts mit einem eine Antenne bildenden, metallischen Instrumenten-Bauteil;
Figur 4 zeigt ein elektrisch leitendes Kopplungselement zum elektrischen Verbinden eines RFID-Speicheretiketts mit einem eine Antenne bildenden, metallischen Instrumenten-Bauteil;
Figur 5 zeigt eine schematische Darstellung eines RFID-Speicheretiketts, das über ein elektrisch leitendes Kopplungselement elektrischen mit einem eine Antenne bildenden, metallischen Instrumenten-Bauteil verbunden ist;
Figur 6 zeigt ein weiteres medizinisches oder dentales Instrument mit einem RFID-Speicheretikett und einem metallischen Instrumenten-Bauteil, das eine Antenne für das RFID-Speicheretikett bildet;
Figur 7A zeigt eine weitere Schnittansicht durch ein RFID-Speicheretikett, das auf einem metallischen Instrumenten-Bauteil, das eine Antenne für das RFID-Speicheretikett bildet, angeordnet ist;
Figur 7B zeigt eine weitere Schnittansicht durch ein RFID-Speicheretikett, das auf einem metallischen Instrumenten-Bauteil, das eine Antenne für das RFID-Speicheretikett bildet, angeordnet ist.

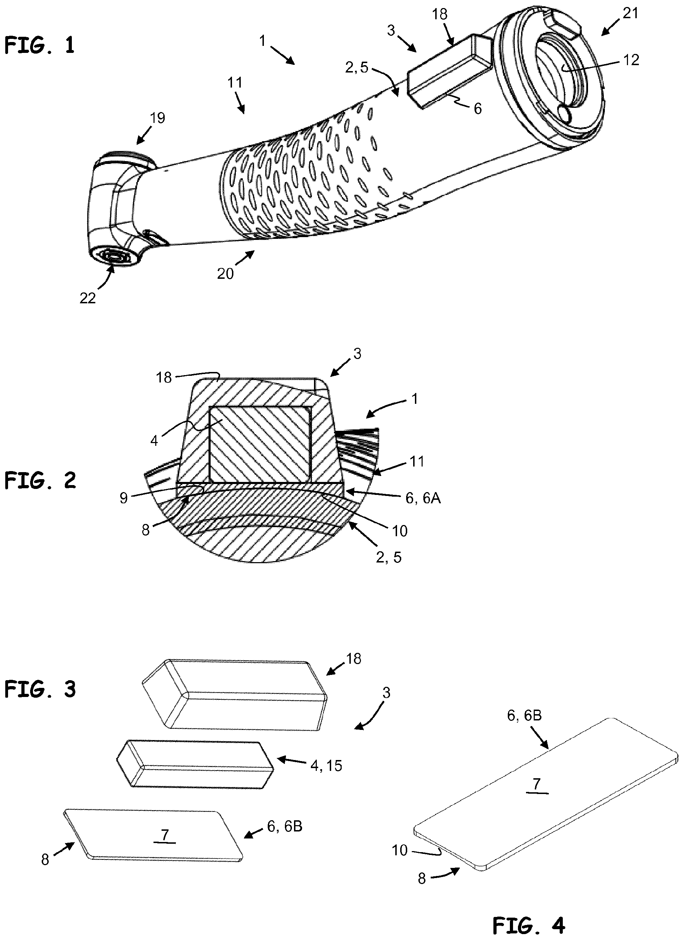

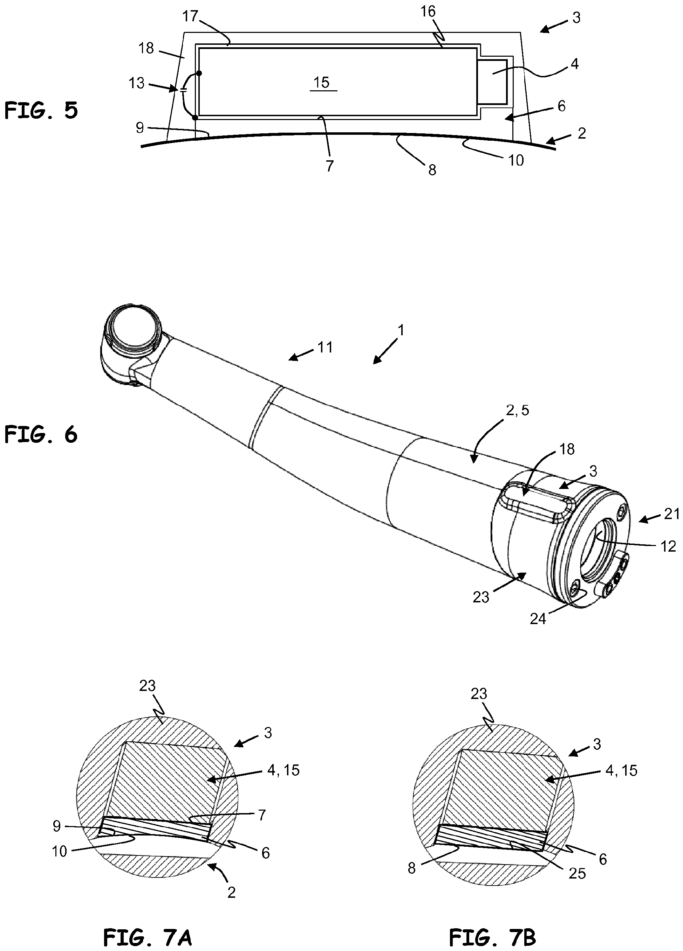

Die Figuren 1 und 6 zeigen jeweils ein medizinisches oder dentales Instrument 1, das als dentales Winkelstück ausgebildet ist. Das Winkelstück 1 umfasst einen Kopfteil 19 und einen daran anschließenden, gebogenen Griffteil 20. Im Kopfteil 19 ist eine Werkzeughalterung 22 beweglich angeordnet, um ein medizinisches oder dentales Behandlungswerkzeug lösbar aufzunehmen. Im Kopfteil 19 und/ oder Griffteil 20 ist des Weiteren eine Antriebsvorrichtung vorgesehen, die zum Beispiel eine oder mehrere Wellen, ein Getriebe, eine Druckgasleitung, ein durch Druckgas antreibbares Laufrad oder ähnliches aufweist. Die Antriebsvorrichtung ist operativ mit der Werkzeughalterung 22 verbunden, um diese und das darin gehaltene Werkzeug in Bewegung zu setzen.

Das medizinisches oder dentales Instrument 1 weist eine Außenhülse 11 auf, die einen am Kopfteil 19 vorgesehenen Kopfhülsenteil und einen am Griffteil 20 vorgesehenen, gebogenen Griffhülsenteil aufweist.

An dem dem Kopfteil 19 abgewandten Ende des medizinischen oder dentalen Instruments 1 ist eine Kupplungsvorrichtung oder Übertragungsvorrichtung 21 vorgesehen, die mit einer Versorgungseinheit oder Dentaleinheit oder einem externen Controller (nicht dargestellt) lösbar verbindbar ist. Die Kupplungsvorrichtung 21 umfasst insbesondere eine Kupplungshülse 12 zur Aufnahme eines Kupplungsgegenstücks, zum Beispiel eines Kupplungsfortsatzes, eines motorischen Antriebs, eines Kupplungselements oder eines Versorgungsschlauchs. Über die Kupplungsvorrichtung 21 ist/ sind zumindest ein Medium, zum Beispiel elektrische Energie, Druckgas, insbesondere Druckluft, Wasser, Licht und/ oder Daten, zum Beispiel Messdaten, Steuer- oder Regeldaten, Identifikationsdaten, zum Beispiel zum Identifizieren eines Werkzeugs, Betriebsdaten oder ähnliche Daten, übertragbar. Die Übertragung des zumindest einen Mediums und/ oder der Daten zwischen dem medizinischen oder dentalen Instrument 1 und der Versorgungseinheit oder Dentaleinheit oder dem externen Controller kann unidirektional oder bidirektional ausgebildet sein.

An dem medizinischen oder dentalen Instrument 1 ist des Weiteren ein RFID-Speicheretikett 3 mit einem Speicherchip 4 zur Speicherung von Daten vorgesehen. Das RFID-Speicheretikett 3 ist derart an dem Winkelstück 1 befestigt, dass das RFID-Speicheretikett 3 mit einem metallischen Instrumenten-Bauteil 2 elektrisch verbunden ist, so dass das metallische Instrumenten-Bauteil 2 eine Antenne 5 für das RFID-Speicheretikett 3 zur drahtlosen Energie- und/ oder Datenübertragung mit einer entfernten Lese- und/ oder Schreibvorrichtung bildet. Das metallische Instrumenten-Bauteil 2, das als Antenne 5 wirkt, ist durch zumindest einen (metallischen) Teil der Außenhülse 11, insbesondere durch zumindest einen Abschnitt des Griffhülsenteils des Griffteils 20 gebildet.

Ein elektrisch leitendes Koppelelement 6 ist dazu vorgesehen, das RFID-Speicheretikett 3 elektrisch mit dem eine Antenne 5 bildenden, metallischen Instrumenten-Bauteil 2, d.h. zumindest einem Abschnitt der Außenhülse 11 zu verbinden. Wie insbesondere aus den Figuren 2 - 5 zu erkennen ist, umfasst das elektrisch leitende Koppelelement 6 eine im Wesentlichen plane, erste Koppelfläche 7, auf welcher das RFID-Speicheretikett 3 angeordnet ist, und eine zweite Koppelfläche 8, welche an dem eine Antenne 5 bildenden, metallischen Instrumenten-Bauteil 2 (zumindest einem Teil der Außenhülse 11) angeordnet ist.

Gemäß einem, insbesondere in den Figuren 2, 5 und 7A dargestellten, Ausführungsbeispiel weist das metallische Instrumenten-Bauteil 2, zum Beispiel ein Abschnitt der Außenhülse 11, eine erste Biegung 9 und die zweite Koppelfläche 8 des elektrisch leitenden Koppelelements 6 eine zur ersten Biegung 9 des metallischen Instrumenten-Bauteils 2 komplementäre zweite Biegung 10 auf, wobei zur elektrischen Verbindung des RFID-Speicheretiketts 3 mit dem eine Antenne 5 bildenden, metallischen Instrumenten-Bauteil 2 die erste Biegung 9 und die zweite Biegung 10 ineinander gefügt sind. Die Verbindung oder der Kontakt zwischen der ersten Biegung 9 und der zweite Biegung 10 erstreckt sich insbesondere über die gesamte zweite Biegung 10 der zweiten Koppelfläche 8 und ist bevorzugt kontinuierlich und/ oder abstandslos. Gemäß den Figuren 2, 5 und 7A ist die erste Biegung 9 konvex und die zweite Biegung 10 konkav geformt, wobei jedoch auch andere Formen denkbar sind.

Das in der Figur 2 dargestellte elektrisch leitende Koppelelement 6 umfasst ein elektrisch leitendes Klebemittel 6A, welches das RFID-Speicheretikett 3 mit dem eine Antenne 5 bildenden, metallischen Instrumenten-Bauteil 2, 11 haftend und elektrisch verbindet. Das RFID-Speicheretikett 3 ist somit insbesondere auf das medizinische oder dentale Instrument 1, vorzugsweise die Außenhülse 11, aufgeklebt. Neben dem bereits beschriebenen Vorteil einer verbesserten Energie- und/ oder Datenübertragung zwischen dem RFID-Speicheretikett 3 und dem eine Antenne 5 bildenden, metallischen Instrumenten-Bauteil 2, 11 aufgrund einer vergrößerten Kopplungsfläche ermöglicht die Verwendung des elektrisch leitenden Klebemittels 6A auch eine besonders einfache und kostengünstige Befestigung des RFID-Speicheretiketts 3 an dem medizinischen oder dentalen Instrument 1, insbesondere an der metallischen Außenhülse 11 oder einem metallischen Abschnitt der Außenhülse 11. Besonders bevorzugt ist damit auch ein unkompliziertes (Verfahren zum) Nachrüsten eines medizinischen oder dentalen Instruments 1 durch Kleben des RFID-Speicheretikett 3 auf das medizinische oder dentale Instrument 1, insbesondere auf die Außenhülse 11 möglich. In dem elektrisch leitenden Klebemittel 6A sind insbesondere elektrisch leitende Partikel (nicht dargestellt) enthalten.

Das in den Figur 3 und 4 dargestellte elektrisch leitende Koppelelement 6 umfasst ein plattenförmiges elektrisch leitendes, insbesondere metallisches oder Metall enthaltendes Formelement 6B. Das Formelement 6B umfasst zwei einander gegenüberliegende Grundflächen welche die erste und zweite Koppelfläche 7, 8 bilden, und mehrere, zum Beispiel vier, Seitenflächen, welche die Koppelflächen 7, 8 verbinden. Die erste Grund- oder Koppelfläche 7, auf welcher das RFID-Speicheretikett 3 angeordnet ist, ist plan. Die zweite Grund- oder Koppelfläche 8, welche das metallische Instrumenten-Bauteil 2 kontaktiert, ist gebogen, insbesondere konkav geformt. Es ist insbesondere in Figur 4 zu erkennen, dass diese zweite Biegung 10 sich von einer der Seitenflächen in Richtung einer Mittellinie der zweiten Grund- oder Koppelfläche 8 erstreckt, so dass die Dicke oder Wandstärke des Formelements 6B in Richtung der Mittellinie abnimmt.

Die Figur 5 zeigt eine schematische Darstellung des Aufbaus und einiger Bestandteile eines RFID-Speicheretiketts 3, das über ein elektrisch leitendes Kopplungselement 6 elektrischen mit einem eine Antenne bildenden, metallischen, gebogenen Instrumenten-Bauteil 2 verbunden ist. Das RFID-Speicheretikett 3 umfasst einen, insbesondere keramischen, Träger 15 für den Speicherchip 4. Auf dem Träger 15 ist zumindest ein mit dem Speicherchip 4 elektrisch verbundener elektrischer Leiter 16, insbesondere in Form einer elektrisch leitenden Beschichtung, vorgesehen, welcher Energie und/ oder Daten zu dem Speicherchip 4 überträgt. Eine elektrisch isolierende Schutzschicht 17, zum Beispiel in Form eines Lacks, umgibt den Träger 15, den Speicherchip 4 und den elektrischen Leiter 16. Der, insbesondere keramische, Träger 15 und der mit dem Speicherchip 4 elektrisch verbundene elektrische Leiter 16 bilden insbesondere einen Antennenabschnitt des RFID-Speicheretiketts 3, der mit dem eine Antenne 5 bildenden, metallischen Instrumenten-Bauteil 2, 11 zur Energie- und/ oder Datenübertragung an die Lese- und/ oder Schreibvorrichtung zusammenwirkt.

Die erste Koppelfläche 7 des elektrisch leitenden Kopplungselement 6 in Figur 5, auf welcher das RFID-Speicheretikett 3 angeordnet ist, ist plan ausgebildet und umfasst zwei durch eine Stufe getrennte ebene Flächen. Die zweite Grund- oder Koppelfläche 8 ist gebogen, insbesondere konkav, geformt und kontaktiert das eine Antenne bildende, konvex gebogene Instrumenten-Bauteil 2.

Eine an dem medizinischen oder dentalen Instrument 1, insbesondere an dem eine Antenne 5 bildenden, metallischen Instrumenten-Bauteil 2 und/ oder der Außenhülse 11 befestigte Kappe 18 umschließt das RFID-Speicheretikett 3 und insbesondere auch das elektrisch leitende Koppelelement 6. Die auch in den Figuren 1 - 3 dargestellte Kappe 18 ist insbesondere derart an dem metallischen Instrumenten-Bauteil 2 befestigt, dass das RFID-Speicheretikett 3 und insbesondere auch das elektrisch leitende Koppelelement 6 dichtend umschlossen sind, um das Eindringen von Fremdstoffen von außen in das RFID-Speicheretikett 3 zu unterbinden.

In der Figur 5 ist zusätzlich schematisch angedeutet, dass das elektrisch leitende Koppelelement 6 mit zumindest Teilen des RFID-Speicheretiketts 3 und des eine Antenne 5 bildenden, metallischen Instrumenten-Bauteils 2 (zumindest eines Teils der Außenhülse 11), insbesondere in einem Ersatzschaltbild, einen elektrischen Kondensator 13 zur kapazitiven Energie- und/ oder Datenübertragung bildet. Die Elektroden des Kondensators 13 sind insbesondere durch das metallische Instrumenten-Bauteil 2, zum Beispiel einen Abschnitt der Außenhülse 11, das elektrisch leitende Koppelelement 6 und elektrisch leitende Elemente des RFID-Speicheretiketts 3, zum Beispiel den mit dem Speicherchip 4 elektrisch verbundenen elektrischen Leiter 16, der insbesondere eine elektrisch leitende Beschichtung aufweist, gebildet. Das Dielektrikum des Kondensators 13 umfasst insbesondere die elektrisch isolierende Schutzschicht 17 des RFID-Speicheretiketts 3, die vorzugsweise zumindest den Speicherchip 4 umgibt.

In der Figur 6 ist das RFID-Speicheretiketts 3 in das medizinische oder dentale Instrument 1, insbesondere in die Außenhülse 11, integriert, so dass es vorzugsweise nur gering über die Außenhülse 11 ragt, bündig mit der Außenhülse 11 abschließt oder vollständig in der Außenhülse 11 aufgenommen ist. Insbesondere ragt das RFID-Speicheretikett 3 nicht so weit über die Außenhülse 11 wie dies bei dem medizinischen oder dentalen Instrument 1 der Figur 1 gezeigt ist. Bevorzugt ist das RFID-Speicheretikett 3 an einem elektrisch isolierenden Bauteil 23 angeordnet, das an dem medizinischen oder dentalen Instrument 1 befestigt oder befestigbar ist. Das elektrisch isolierende Bauteil 23 ist zum Beispiel aus Keramik oder Kunststoff gefertigt, um insbesondere eine ungestörte Energie- und/ oder Datenübertragung zu gewährleisten. Das elektrisch isolierende Bauteil 23 umfasst zum Beispiel einen Ring oder ein bogenförmiges Element, das insbesondere auf ein Bauteil des medizinischen oder dentalen Instruments 1 aufschiebbar ist. Das ring- der bogenförmige Bauteil 23 ist zum Beispiel über die Kupplungshülse 12 der Kupplungsvorrichtung 21 schiebbar und insbesondere über eine oder mehrere Schrauben 24 an dem medizinischen oder dentalen Instrument 1 befestigbar.

Die Figur 7A zeigt ein mögliches Ausführungsbeispiel eines in das medizinische oder dentale Instrument 1, insbesondere in die Außenhülse 11, integrierten RFID-Speicheretiketts 3. Wie unter Bezug auf die vorstehenden Ausführungsbeispiele beschrieben weist das metallische Instrumenten-Bauteil 2, welches hier zum Beispiel durch zumindest einen Teil der Kupplungshülse 12 und/ oder der Außenhülse 11 gebildet ist, eine erste Biegung 9 und die zweite Koppelfläche 8 des elektrisch leitenden Koppelelements 6 eine zur ersten Biegung 9 des metallischen Instrumenten-Bauteils 2 komplementäre zweite Biegung 10 auf. Zur elektrischen Verbindung des RFID-Speicheretiketts 3 mit dem eine Antenne 5 bildenden, metallischen Instrumenten-Bauteil 2 sind die erste Biegung 9 und die zweite Biegung 10 ineinandergefügt. Das elektrisch leitende Koppelelement 6 kann sowohl als elektrisch leitendes Klebemittel 6A oder als Formelement 6B ausgeführt sein, so wie dies im Vorstehenden beschrieben ist.

Die Figur 7B zeigt ein alternatives Ausführungsbeispiel eines in das medizinische oder dentale Instrument 1, insbesondere in die Außenhülse 11, integrierten RFID-Speicheretiketts 3. Hier weist das metallische Instrumenten-Bauteil 2, welches zum Beispiel durch zumindest einen Teil der Kupplungshülse 12 und/ oder der Außenhülse 11 gebildet ist, eine Planfläche 25 auf, an welcher das RFID-Speicheretikett 3 angeordnet ist. Demgemäß ist auch die zweite Koppelfläche 8 des elektrisch leitenden Koppelelements 6 plan ausgebildet. Zur elektrischen Verbindung des RFID-Speicheretiketts 3 mit dem eine Antenne 5 bildenden, metallischen Instrumenten-Bauteil 2 kontaktieren die Planfläche 25 und die plane zweite Koppelfläche 8 einander. Das elektrisch leitende Koppelelement 6 kann sowohl als elektrisch leitendes Klebemittel 6A oder als Formelement 6B ausgeführt sein, so wie dies im Vorstehenden beschrieben ist.

Die beschriebenen oder dargestellten Ausführungsbeispiele dienen insbesondere der Veranschaulichung der Erfindung. Die in einem Ausführungsbeispiel offenbarten Merkmale sind daher nicht auf dieses Ausführungsbeispiel beschränkt, sondern sind einzeln oder gemeinsam mit einem oder mehreren Merkmalen eines der anderen Ausführungsbeispiele kombinierbar.

## Patentansprüche

1. Medizinisches oder dentales Instrument (1), umfassend: ein metallisches Instrumenten-Bauteil (2), ein RFID-Speicheretikett (3) mit einem Speicherchip (4) zur Speicherung von Daten, wobei das RFID-Speicheretikett (3) derart an dem medizinischen oder dentalen Instrument (1) befestigt ist, dass das RFID-Speicheretikett (3) mit dem metallischen Instrumenten-Bauteil (2) elektrisch verbunden ist, so dass das metallische Instrumenten-Bauteil (2) eine Antenne (5) für das RFID-Speicheretikett (3) zur drahtlosen Energie- und/ oder Datenübertragung mit einer entfernten Lese- und/ oder Schreibvorrichtung bildet, und
ein elektrisch leitendes Koppelelement (6), welches das RFID-Speicheretikett (3) elektrisch mit dem eine Antenne (5) bildenden, metallischen Instrumenten-Bauteil (2) verbindet, wobei das elektrisch leitende Koppelelement (6) eine im Wesentlichen plane, erste Koppelfläche (7), auf welcher das RFID-Speicheretikett (3) angeordnet ist, und eine zweite Koppelfläche (8) aufweist, welche an dem eine Antenne (5) bildenden, metallischen Instrumenten-Bauteil (2) angeordnet ist, **dadurch gekennzeichnet, dass**
das elektrisch leitende Koppelelement (6) mit zumindest Teilen des RFID-Speicheretiketts (3) und des eine Antenne (5) bildenden, metallischen Instrumenten-Bauteils (2) einen elektrischen Kondensator (13) zur kapazitiven Energie- und/ oder Datenübertragung zwischen dem RFID-Speicheretikett (3) und dem eine Antenne (5) bildenden, metallischen Instrumenten-Bauteils (2) bildet.

2. Medizinisches oder dentales Instrument (1) nach Anspruch 1, **dadurch gekennzeichnet, dass**,
das metallische Instrumenten-Bauteil (2) eine erste Biegung (9) aufweist und die zweite Koppelfläche (8) des elektrisch leitenden Koppelelements (6) eine zur ersten Biegung (9) des metallischen Instrumenten-Bauteils (2) komplementäre zweite Biegung (10) aufweist, wobei zur elektrischen Verbindung des RFID-Speicheretiketts (3) mit dem eine Antenne (5) bildenden, metallischen Instrumenten-Bauteil (2) die erste Biegung (9) und die zweite Biegung (10) ineinandergefügt sind.

3. Medizinisches oder dentales Instrument (1) nach Anspruch 1 oder 2, **dadurch gekennzeichnet, dass**
das metallische Instrumenten-Bauteil (2) zylindrisch oder hohl-zylindrisch ausgebildet ist.

4. Medizinisches oder dentales Instrument (1) nach einem der vorstehenden Ansprüche, **dadurch gekennzeichnet, dass**
das metallische Instrumenten-Bauteil (2) hülsenförmig ausgebildet ist und zumindest einen Abschnitt einer Griffhülse oder einer Außenhülse (11) oder einer Lagerhülse für ein Element des medizinischen oder dentalen Instruments (1) oder einer Kupplungshülse (12) zur lösbaren Verbindung des medizinischen oder dentalen Instruments (1) mit einem Kupplungsteil umfasst.

5. Medizinisches oder dentales Instrument (1) nach einem der vorstehenden Ansprüche, **dadurch gekennzeichnet, dass**
Elektroden des Kondensators (13) durch das metallische Instrumenten-Bauteil (2), das elektrisch leitende Koppelelement (6) und elektrisch leitende Elemente des RFID-Speicheretiketts (3) gebildet sind.

6. Medizinisches oder dentales Instrument (1) nach einem der vorstehenden Ansprüche, **dadurch gekennzeichnet, dass**
ein Dielektrikum des Kondensators (13) eine elektrisch isolierende Schutzschicht (17) des RFID-Speicheretiketts (3) umfasst.

7. Medizinisches oder dentales Instrument (1) nach einem der vorstehenden Ansprüche, **dadurch gekennzeichnet, dass**
das elektrisch leitende Koppelelement (6) ein elektrisch leitendes Klebemittel (6A) umfasst, welches das RFID-Speicheretikett (3) mit dem eine Antenne (5) bildenden, metallischen Instrumenten-Bauteil (2) haftend und elektrisch verbindet.

8. Medizinisches oder dentales Instrument (1) nach Anspruch 7, **dadurch gekennzeichnet, dass**
das Klebemittel (6A) elektrisch leitende Partikel aufweist.

9. Medizinisches oder dentales Instrument (1) nach einem der vorstehenden Ansprüche, **dadurch gekennzeichnet, dass**
das elektrisch leitende Koppelelement (6) ein elektrisch leitendes Formelement (6B) aufweist.

10. Medizinisches oder dentales Instrument (1) nach Anspruch 9, **dadurch gekennzeichnet, dass**
das elektrisch leitende Formelement (6B) durch ein vollständig metallisches Formelement oder durch ein Trägermaterial mit metallischen Partikeln gebildet ist.

11. Medizinisches oder dentales Instrument (1) nach einem der vorstehenden Ansprüche, **dadurch gekennzeichnet, dass**
das elektrisch leitende Koppelelement (6) plattenförmig ausgebildet ist und/ oder zumindest eine gewölbte Fläche (14) aufweist.

12. Medizinisches oder dentales Instrument (1) nach einem der vorstehenden Ansprüche, **dadurch gekennzeichnet, dass**
das RFID-Speicheretikett (3) des Weiteren aufweist: einen Träger (15) für den Speicherchip (4); zumindest einen mit dem Speicherchip (4) elektrisch verbundenen elektrischen Leiter (16); eine elektrisch isolierende Schutzschicht (17).

13. Medizinisches oder dentales Instrument (1) nach einem der vorstehenden Ansprüche, **gekennzeichnet durch**
eine an dem medizinischen oder dentalen Instrument (1) befestigte Kappe (18), welche das RFID-Speicheretikett (3) und insbesondere auch das elektrisch leitende Koppelelement (6) umschließt.

14. Verfahren zur Herstellung oder zum Nachrüsten eines medizinischen oder dentalen Instruments (1), **gekennzeichnet durch**,
zur Verfügung Stellen eines medizinischen oder dentalen Instruments (1) mit einem metallischen Instrumenten-Bauteil (2),
zur Verfügung Stellen eines RFID-Speicheretiketts (3) mit einem Speicherchip (4) zur Speicherung von Daten,
Befestigen des RFID-Speicheretiketts (3) an dem medizinischen oder dentalen Instrument (1) mittels eines elektrisch leitenden Koppelelements (6), derart, dass das RFID-Speicheretikett (3) mit dem metallischen Instrumenten-Bauteil (2) elektrisch verbunden ist, so dass das metallische Instrumenten-Bauteil (2) eine Antenne (5) für das RFID-Speicheretikett (3) zur drahtlosen Energie- und/ oder Datenübertragung mit einer entfernten Lese- und/ oder Schreibvorrichtung bildet, wobei das elektrisch leitende Koppelelement (6), eine im Wesentlichen plane, erste Koppelfläche (7), auf welcher das RFID-Speicheretikett (3) angeordnet ist, und eine zweite Koppelfläche (8) aufweist, welche an dem eine Antenne (5) bildenden, metallischen Instrumenten-Bauteil (2) angeordnet ist, und wobei das elektrisch leitende Koppelelement (6) mit zumindest Teilen des RFID-Speicheretiketts (3) und des eine Antenne (5) bildenden, metallischen Instrumenten-Bauteils (2) einen elektrischen Kondensator (13) zur kapazitiven Energie- und/ oder Datenübertragung bildet.

15. Verfahren zur Herstellung oder zum Nachrüsten eines medizinischen oder dentalen Instruments, nach Anspruch 14, **dadurch gekennzeichnet, dass** das metallische Instrumenten-Bauteil (2) eine erste Biegung (9) aufweist und die zweite Koppelfläche (8) des elektrisch leitenden Koppelelements (6) eine zur ersten Biegung (9) des metallischen Instrumenten-Bauteils (2) komplementäre zweite Biegung (10) aufweist, wobei zur elektrischen Verbindung des RFID-Speicheretiketts (3) mit dem eine Antenne (5) bildenden, metallischen Instrumenten-Bauteil (2) die erste Biegung (9) und die zweite Biegung (10) ineinandergefügt werden.

## Claims

1. A medical or dental instrument (1) comprising: a metal instrument component (2), an RFID memory tag (3) having a memory chip (4) for storing data, wherein the RFID memory tag (3) is attached to the medical or dental instrument (1) such that the RFID memory tag (3) is electrically connected to the metal instrument component (2) such that the metal instrument component (2) forms an antenna (5) for the RFID memory tag (3) for wireless energy and/or data transfer to/from a remote reading and/or writing device, and
an electrically conductive coupling element (6) electrically connecting the RFID memory tag (3) to the metal instrument component (2) forming an antenna (5), the electrically conductive coupling element (6) comprising a substantially planar first coupling surface (7) on which the RFID memory tag (3) is arranged and a second coupling surface (8) arranged on the metal instrument component (2) forming the antenna (5), **characterized in that**
the electrically conductive coupling element (6) forms with at least parts of the RFID memory tag (3) and the metal instrument component (2) forming an antenna (5) an electrical capacitor (13) for capacitive energy and/or data transfer between the RFID memory tag (3) and the metal instrument component (2) forming an antenna (5).

2. The medical or dental instrument (1) according to claim 1, **characterized in that** the metal instrument component (2) comprises a first bend (9) and the second coupling surface (8) of the electrically conductive coupling element (6) comprises a second bend (9) complementary to the first bend (10) of the metal instrument component (2), wherein the first bend (9) and the second bend (10) are interlocked to electrically connect the RFID memory tag (3) to the metal instrument component (2) forming an antenna (5).

3. The medical or dental instrument (1) according to claim 1 or 2, **characterized in that** the metal instrument component (2) has a cylindrical or hollow-cylindrical design.

4. The medical or dental instrument (1) according to any one of the preceding claims, **characterized in that**
the metal instrument component (2) has a sleeve-like design and comprises at least a portion of a grip sleeve or an outer shell (11) or a bearing sleeve for an element of the medical or dental instrument (1) or a coupling sleeve (12) for detachably connecting the medical or dental instrument (1) to a coupling part.

5. The medical or dental instrument (1) according to any one of the preceding claims, **characterized in that**
electrodes of the capacitor (13) are formed by the metal instrument component (2), the electrically conductive coupling element (6), and electrically conductive elements of the RFID memory tag (3).

6. The medical or dental instrument (1) according to any one of the preceding claims, **characterized in that**
a dielectric of the capacitor (13) comprises an electrically insulating protective layer (17) of the RFID memory tag (3).

7. The medical or dental instrument (1) according to any one of the preceding claims, **characterized in that**
the electrically conductive coupling element (6) comprises an electrically conductive adhesive (6A) which affixes and electrically connects the RFID memory tag (3) to the metal instrument component (2) forming an antenna (5).

8. The medical or dental instrument (1) according to claim 7, **characterized in that** the adhesive (6A) comprises electrically conductive particles.

9. The medical or dental instrument (1) according to any one of the preceding claims, **characterized in that**
the electrically conductive coupling element (6) comprises an electrically conductive shaped element (6B).

10. The medical or dental instrument (1) according to claim 9, **characterized in that** the electrically conductive shaped element (6B) is formed by a completely metal shaped element or by a carrier material comprising metal particles.

11. The medical or dental instrument (1) according to any one of the preceding claims, **characterized in that**
the electrically conductive coupling element (6) is plate-shaped and/or comprises at least one curved surface (14).

12. The medical or dental instrument (1) according to any one of the preceding claims, **characterized in that**
the RFID memory tag (3) further comprises: a carrier (15) for the memory chip (4); at least one electrical conductor (16) electrically connected to the memory chip (4); an electrically insulating protective layer (17).

13. The medical or dental instrument (1) according to any one of the preceding claims, **characterized by**
a cap (18) which is fastened to the medical or dental instrument (1) and encloses the RFID memory tag (3) and in particular also the electrically conductive coupling element (6).

14. A method for manufacturing or retrofitting a medical or dental instrument (1), **characterized by**
providing a medical or dental instrument (1) comprising a metal instrument component (2),
providing an RFID memory tag (3) comprising a memory chip (4) for storing data, attaching the RFID memory tag (3) to the medical or dental instrument (1) through an electrically conductive coupling element (6), such that the RFID memory tag (3) is electrically connected to the metal instrument component (2), so that the metal instrument component (2) forms an antenna (5) for the RFID memory tag (3) for wireless energy and/or data transfer to/from a remote reading and/or writing device, wherein the electrically conductive coupling element (6) comprises an substantially planar first coupling surface (7) on which the RFID memory tag (3) is arranged and a second coupling surface (8) arranged on the metal instrument component (2) which forms an antenna (5) and wherein the electrically conductive coupling element (6) forms with at least parts of the RFID memory tag (3) and the metal instrument component (2) forming an antenna (5) an electrical capacitor (13) for capacitive energy and/or data transfer.

15. The method for manufacturing or retrofitting a medical or dental instrument, according to claim 14, **characterized in that**
the metal instrument component (2) comprises a first bend (9) and the second coupling surface (8) of the electrically conductive coupling element (6) comprises a second bend (9) complementary to the first bend (10) of the metal instrument component (2), wherein the first bend (9) and the second bend (10) are interlocked to electrically connect the RFID memory tag (3) to the metal instrument component (2) forming an antenna (5).

## Revendications

1. Instrument médical ou dentaire (1), comprenant: un composant d'instrument métallique (2), une étiquette mémoire RFID (3) avec une puce de mémoire (4) pour le stockage de données, dans lequel l'étiquette mémoire RFID (3) est fixée à l'instrument médical ou dentaire (1) de telle sorte que l'étiquette mémoire RFID (3) est raccordée électriquement au composant d'instrument métallique (2) de sorte que le composant d'instrument métallique (2) forme une antenne (5) pour l'étiquette mémoire RFID (3) pour la transmission sans fil d'énergie et/ou de données avec un dispositif de lecture et/ou écriture distant, et
un élément de couplage électroconducteur (6) qui raccorde électriquement l'étiquette mémoire RFID (3) au composant d'instrument métallique (2) formant une antenne (5), dans lequel l'élément de couplage électroconducteur (6) présente une première surface de couplage (7) sensiblement plane sur laquelle l'étiquette mémoire RFID (3) est disposée et une deuxième surface de couplage (8) disposée au niveau du composant d'instrument métallique (2) formant une antenne (5), **caractérisé en ce que**
l'élément de couplage électroconducteur (6) forme, avec au moins des parties de l'étiquette mémoire RFID (3) et le composant d'instrument métallique (2) formant une antenne (5), un condensateur électrique (13) pour la transmission capacitive d'énergie et/ou de données entre l'étiquette mémoire RFID (3) et le composant d'instrument métallique (2) formant une antenne (5).

2. Instrument médical ou dentaire (1) selon la revendication 1, **caractérisé en ce que**
le composant d'instrument métallique (2) présente une première courbure (9) et la deuxième surface de couplage (8) de l'élément de couplage électroconducteur (6) présente une deuxième courbure (10) complémentaire à la première courbure (9) du composant d'instrument métallique (2), dans lequel, pour le raccordement électrique de l'étiquette mémoire RFID (3) au composant d'instrument métallique (2) formant une antenne (5), la première courbure (9) et la deuxième courbure (10) sont emboîtées.

3. Instrument médical ou dentaire (1) selon la revendication 1 ou 2, **caractérisé en ce que**
le composant d'instrument métallique (2) est réalisé de manière cylindrique ou cylindrique creuse.

4. Instrument médical ou dentaire (1) selon l'une des revendications précédentes, **caractérisé en ce que**
le composant d'instrument métallique (2) est réalisé en forme de manchon et comprend au moins une partie d'un manchon de poignée ou d'un manchon extérieur (11) ou d'un manchon de palier pour un élément de l'instrument médical ou dentaire (1) ou d'un manchon d'accouplement (12) pour la liaison amovible entre l'instrument médical ou dentaire (1) et une partie de couplage.

5. Instrument médical ou dentaire (1) selon l'une des revendications précédentes, **caractérisé en ce que**
des électrodes du condensateur (13) sont formées par le composant d'instrument métallique (2), l'élément de couplage électroconducteur (6), et des éléments électroconducteurs de l'étiquette mémoire RFID (3).

6. Instrument médical ou dentaire (1) selon l'une des revendications précédentes, **caractérisé en ce qu'**
un diélectrique du condensateur (13) comprend une couche de protection d'isolation électrique (17) de l'étiquette mémoire RFID (3).

7. Instrument médical ou dentaire (1) selon l'une des revendications précédentes, **caractérisé en ce que**
l'élément de couplage électroconducteur (6) comprend un adhésif électroconducteur (6A) qui raccorde électriquement et par adhérence l'étiquette mémoire RFID (3) au composant d'instrument métallique (2) formant une antenne (5).

8. Instrument médical ou dentaire (1) selon la revendication 7, **caractérisé en ce que** l'adhésif (6A) présente des particules électroconductrices.

9. Instrument médical ou dentaire (1) selon l'une des revendications précédentes, **caractérisé en ce que**
l'élément de couplage électroconducteur (6) présente un élément formé électroconducteur (6B).

10. Instrument médical ou dentaire (1) selon la revendication 9, **caractérisé en ce que** l'élément formé électroconducteur (6B) est formé par un élément formé entièrement métallique ou par un matériau de support avec des particules métalliques.

11. Instrument médical ou dentaire (1) selon l'une des revendications précédentes, **caractérisé en ce que**
l'élément de couplage électroconducteur (6) est réalisé en forme de plaque et/ou présente au moins une surface bombée (14).

12. Instrument médical ou dentaire (1) selon l'une des revendications précédentes, **caractérisé en ce que**
l'étiquette mémoire RFID (3) présente en outre: un support (15) pour la puce de mémoire (4); au moins un conducteur électrique (16) raccordé électriquement à la puce de mémoire (4); une couche de protection d'isolation électrique (17).

13. Instrument médical ou dentaire (1) selon l'une des revendications précédentes, **caractérisé par**
un capuchon (18) fixé sur l'instrument médical ou dentaire (1), lequel renferme l'étiquette mémoire RFID (3) et en particulier également l'élément de couplage électroconducteur (6).

14. Procédé pour la fabrication ou le renouvellement d'un instrument médical ou dentaire (1), **caractérisé par**
la mise à disposition d'un instrument médical ou dentaire (1) avec un composant d'instrument métallique (2),
la mise à disposition d'une étiquette mémoire RFID (3) avec une puce de mémoire (4) pour le stockage de données,
la fixation de l'étiquette mémoire RFID (3) à l'instrument médical ou dentaire (1) au moyen d'un élément de couplage électroconducteur (6) de telle sorte que l'étiquette mémoire RFID (3) est raccordée électriquement au composant d'instrument métallique (2) de sorte que le composant d'instrument métallique (2) forme une antenne (5) pour l'étiquette mémoire RFID (3) pour la transmission sans fil d'énergie et/ou de données avec un dispositif de lecture et/ou écriture distant, dans lequel l'élément de couplage électroconducteur (6) présente une première surface de couplage sensiblement plane (7) sur laquelle l'étiquette mémoire RFID (3) est disposée et une deuxième surface de couplage (8) disposée au niveau du composant d'instrument métallique (2) formant une antenne (5), et dans lequel l'élément de couplage électroconducteur (6) forme, avec au moins des parties de l'étiquette mémoire RFID (3) et le composant d'instrument métallique (2) formant une antenne (5), un condensateur électrique (13) pour la transmission capacitive d'énergie et/ou de données.

15. Procédé pour la fabrication ou le renouvellement d'un instrument médical ou dentaire selon la revendication 14, **caractérisé en ce que**
le composant d'instrument métallique (2) présente une première courbure (9) et la deuxième surface de couplage (8) de l'élément de couplage électroconducteur (6) présente une deuxième courbure (10) complémentaire à la première courbure (9) du composant d'instrument métallique (2), dans lequel, pour le raccordement électrique de l'étiquette mémoire RFID (3) au composant d'instrument métallique (2) formant une antenne (5), la première courbure (9) et la deuxième courbure (10) sont emboîtées.
